# EUROPÄISCHE PATENTSCHRIFT

(11) **EP 2 249 725 B1**
(45) Veröffentlichungstag und Bekanntmachung des Hinweises auf die Patenterteilung: **10.06.2015**
(21) Anmeldenummer: 09736551.4
(22) Anmeldetag: 19.02.2009
(51) Int. Cl.: A61B 17/70, A61B 17/88

(54) **Vorrichtung zur Stabilisierung der Wirbelsäule**
Device for stabilizing the spinal column
Dispositif de stabilisation de la colonne vertébrale

(43) Veröffentlichungstag der Anmeldung: 17.11.2010
(73) Patentinhaber: Ulrich GmbH & Co. KG, 89081 Ulm (DE)
(72) Erfinder: WERNER, Claudia, 89233 Neu-Ulm (DE)
(74) Vertreter: Hentrich Patentanwälte PartG mbB
(86) Internationale Anmeldenummer: PCT/DE2009/075005
(87) Internationale Veröffentlichungsnummer: WO 2010/094250

(56) Entgegenhaltungen:
- WO-A-97/43974
- WO-A-2004/082522
- US-A- 5 989 250
- US-A- 6 123 706
- US-A- 6 123 707
- US-A1- 2003 088 248
- US-A1- 2005 096 653
- US-A1- 2006 149 231
- US-B1- 6 613 050

## Beschreibung

Die Erfindung betrifft eine Vorrichtung zur Stabilisierung der Wirbelsäule, mit einer einen Schraubenschaft aufweisenden Knochenschraube, die einen zum Einbringen in den Knochen aufweisenden ersten Gewindeabschnitt aufweist, mit einem Stab und mit einem an der Knochenschraube befestigbaren Verbinder zum lösbaren Verbinden des Stabes mit der Knochenschraube, wobei der Verbinder einen Verbinderkörper mit einer Durchgangsöffnung und einer einseitig offenen Stabaufnahme aufweist, die durch zwei einander gegenüberliegende, zur Bildung eines Rastsitzes jeweils mindestens eine Stegleiste aufweisende Körperstege gebildet ist.

Bei traumatischen oder degenerativen Erkrankungen der Wirbelsäule ergibt sich oftmals das Erfordernis, zur Wiederherstellung und Gewährleistung der tragenden Funktion der Wirbelsäule diese mittels einer Instrumentierung zu stabilisieren. Als Beispiel für eine derartige Instrumentierung kann das in der EP 1 254 639 A1 gezeigte Stab-Schraubensystem genannt werden, das Knochenschrauben umfasst, die in die Wirbelkörper eingebracht werden können als Verankerungsträger für Verbinder, die eine Ringöse aufweisen, durch die der Stab durchgeschoben werden kann. Dieser Stab wird benutzt, um mehrere Wirbelkörper zu einer stabilen Einheit zusammenzufassen, Knochenschrauben hindurchgeführt werden muss. Da zunächst die Knochenschrauben in den Wirbelkörper zu verankern, erfordert das nachträgliche Einführen des Stabes durch die Ringösen der Verbinder einen großen operativen Zugang.

Eine Vorrichtung der eingangs genannten Art ist aus der US 6 123 706 bekannt. Bei der ersten Ausführungsform ist der Zugang zur Stabaufnahme durch die den Verbinder durchsetzende Knochenschraube blockiert, so dass der Stab in die Stabaufnahme in seiner Längsrichtung einzuschieben ist. In einer Alternative ist die Lage von Knochenschraube und Stabaufnahme bezüglich des Verbinders vertauscht; gleichwohl ist der Stab in die Stabaufnahme einzuschieben.

Der Erfindung liegt die Aufgabe zugrunde, eine Vorrichtung zur Stabilisierung der Wirbelsäule der eingangs genannten Art so auszubilden, dass die Handhabung während der Operation vereinfacht ist.

Diese Aufgabe wird nach der Erfindung bei einer Vorrichtung der eingangs genannten Art dadurch gelöst, dass die Stegleisten (16) die seitliche Öffnungsweite der Stabaufnahme (11) begrenzen derart, dass beim Einsclipsen des Stabes (3) in die Stabaufnahme (11) unter Ausnutzung einer Federwirkung zunächst eine Aufweitung der Stabaufnahme (11) mittels einer Auslenkung der Körperstege (14) erfolgt, und dass infolge der gewünschten Federwirkung die Körperstege (14) den Stab (3) nach der Einführung fest umschließen und einen an die Umfanggestalt des Stabes (3) angepassten Rastsitz bilden.

Mit einer derartigen Gestaltung des Verbinders eines Stab-Schraubensystems ist der große Vorteil verbunden, dass nicht der Stab einzeln mit einer Bewegung in Richtung seiner Längsachse in die einzelnen Ringösen der verwendeten Verbinder eingeführt werden muss, sondern in einer seitlichen, in radialer Richtung zu der Stablängsachse erfolgenden Bewegung die Positionierung des Stabes an der Knochenschraube mittels des Verbinders erfolgen kann.

Um die Einführung des Stabes in die Rastaufnahme zu vereinfachen, ist im Rahmen der Erfindung weiterhin vorgesehen, dass die Körperstege an zwei mit gegenseitigem Abstand an einer Körperbasis angeschlossenen Körperschenkeln ausgebildet sind. Durch diese Gestaltung wird der Vorteil erreicht, dass für den Verbinderkörper eine große Materialstärke mit entsprechend hoher Belastbarkeit bereitgestellt werden kann, ohne die Auslenkbarkeit der Körperstege zu beeinträchtigen, da die Auslenkbarkeit der Körperstege und der Körperschenkel kumulativ ausgenutzt werden kann.

Wenn die Durchgangsöffnung als Langloch im Verbinderkörper ausgebildet ist, besteht die Möglichkeit einer verbesserten Justierungsmöglichkeit des Verbinders und damit des Stabes relativ zu der Knochenschraube. Dabei ist dann bevorzugt, dass das Langloch in dem Körperschenkel ausgebildet ist und geneigt zur Längsachse des Verbinders verlaufende Seitenwände aufweist, die dann als Anschläge für die Knochenschraube bei der Begrenzung des Verstellwinkels dienen.

Ganz besonders bevorzugt im Rahmen der Erfindung ist es, wenn der Verbinder als Koppelglied zum Anschließen eines Repositeurs ausgebildet ist. Im Rahmen einer Wirbelsäulenoperation ergibt sich nämlich oftmals das Erfordernis, dass nicht nur die Wirbelkörper als Teil der Wirbelsäule einer Stabilisierung bedürfen, sondern auch eine Ausrichtung der Wirbelkörper relativ zueinander erfolgen muss, um die gewünschte natürliche anatomische Krümmung der Wirbelsäule nachzubilden oder eine seitliche Verkrümmung zu korrigieren. Den Verbinder als Koppelglied zum Anschließen eines Repositeurs auszubilden bieten den Vorteil, dass die bereits in den Wirbelkörpern platzierten Knochenschrauben genutzt werden können, um unter Vermittlung des Verbinders durch den Repositeur die Ausrichtung der Wirbelkörper zu bewirken, wobei infolge des Stab-Schraubensystems diese so erzielte Ausrichtung dauerhaft fixiert werden kann, so dass ein sehr einfaches System mit wenigen Bauteilen zur Verfügung gestellt ist mit dem mehrfachen Nutzen einer Verwendbarkeit des Verbinders während der Operation als Koppelglied zum Repositeur und als Teil des Stab-Schraubensystems in der Vorrichtung zur Stabilisierung.

Unter Berücksichtigung der Verdrehbarkeit des Verbinders um seine Längsachse durch das im Verbinderkörper ausgebildeten Langloches ist es zweckmäßig, wenn die Strukturen zum Anschließen des Repositeurs die entsprechende Drehbarkeit des Verbinderkörpers nicht beeinträchtigen. Dies wird erzielt, indem der Verbinder am Verbinderkörper eine in Umfangsrichtung verlaufende Ringnut zum Zusammenwirken mit dem Repositeur aufweist.

Vorteilhaft ist es weiterhin, wenn die Knochenschraube benachbart zum ersten Gewindeabschnitt einen Schaftbund aufweist, da dieser Schaftbund genutzt werden kann, um dem Verbinder eine bestimmte Lage hinsichtlich der axialen Erstreckung der Knochenschraube zuzuweisen, es also nicht erforderlich ist, dass der Verbinder sich selber unmittelbar am Wirbelkörper abstützt.

Im Rahmen der Erfindung ist es weiterhin vorgesehen, dass am Schraubenschaft ein zweiter Gewindeabschnitt ausgebildet ist, und dass ein Fixiermittel zum Zusammenwirken mit dem zweiten Gewindeabschnitt die Vorrichtung zur Stabilisierung der Wirbelsäule ergänzt, da infolge des Zusammenwirkens der Fixiermutter mit dem zweiten Gewindeabschnitt eine Festlegung der Lage des Verbinders relativ zu der Knochenschraube sowie eine Pressung des Stabes in der Stabaufnahme erfolgen kann. Einer verbesserten Kraftübertragung dient dabei, dass eine Unterlegscheibe mit einer in die Umfangsgestalt des Verbinderkörpers angepassten einen Seite und einer zur Anlage an der Fixiermutter bestimmten anderen Seite vorgesehen ist.

Als vorteilhaft hat sich im Rahmen der Erfindung weiterhin erwiesen, wenn der Schraubenschaft im Bereich des zweiten Gewindeabschnitts mindestens einen den Schraubenschaft in Schaftstege teilenden Schlitz aufweist, da so in einfacher Weise die Möglichkeit besteht, den zweiten Gewindeabschnitt der Knochenschraube entsprechend den Erfordernissen zu kürzen, um so weit über die Fixiermutter herausstehende Bereiche der Knochenschraube zu vermeiden. Alternativ oder auch ergänzend besteht die Möglichkeit, dass im Bereich des zweiten Gewindeabschnitts mindestens eine Sollbruchstelle ausgebildet ist.

Im Rahmen der Erfindung ist es wünschenswert, wenn auch unabhängig von einer durch die Fixiermutter auf den Verbinderkörper ausgeübten Kraft bereits eine vorläufige Sicherung des Stabes in der Stabaufnahme gegeben ist, so dass zweckmäßigerweise die Oberfläche der Stabaufnahme und/oder des Stabes die Haltewirkung erhöhende Strukturen und/oder Beschichtungen aufweist. Entsprechenden Strukturen können dabei beispielsweise gebildet sein durch Längs-, Quer- oder Kreuzriefen, die durch eine erhöhte Flächenpressung bzw. entsprechende Kanten oder Noppen ein Eingreifen der einander anliegenden Oberflächen oder zumindest einen Formschluß bewirken.

Die Erfindung betrifft weiterhin eine Kombination einer Vorrichtung zur Stabilisierung der Wirbelsäule nach einem der Ansprüche 1 bis 13, bestehend aus einer Knochenschraube, einem Stab und einem Verbinder mit einem Repositeur, wobei der Verbinder als Koppelglied zum Anschließen des Repositeurs ausgebildet ist, indem der Verbinder am Verbinderkörper eine in Umfangsrichtung verlaufende Ringnut zum Zusammenwirken mit einer einenends offenen Nut des Repositeurs aufweist,

Im folgenden wird die Erfindung an einem in der Zeichnung dargestellten Ausführungsbeispiel näher erläutert; es zeigen:

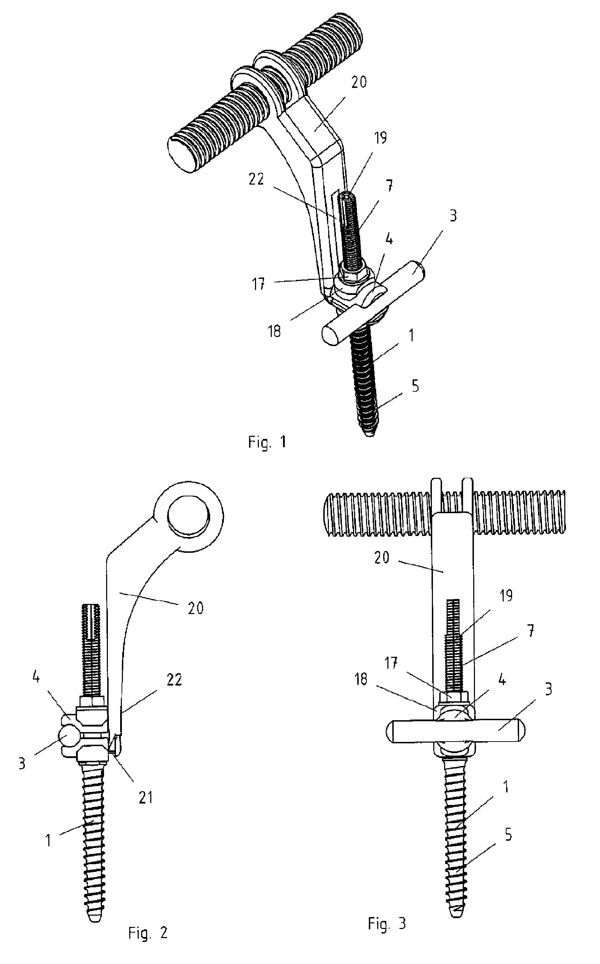

- Fig. 1: eine perspektivische Darstellung der erfindungsgemäßen Vorrichtung,
- Fig. 2: eine Seitenansicht der Vorrichtung aus Figur 1,
- Fig. 3: eine Vorderansicht der Vorrichtung aus Figur 1,
- Fig. 4: eine Seitenansicht des Stab-Schraubensystems nach Entfernung Repositeurs,
- Fig. 5: den Schnitt V - V aus Figur 4,
- Fig. 6: eine Vorderansicht des Stab-Schrauben-Systems aus Figur 4 mit gegenüber der Schraubenlängsachse verdrehtem Verbinder,
- Fig. 7: eine der Figur 6 entsprechende Darstellung mit gegenüber der Schraubenlängsachse in die andere Richtung verdrehtem Verbinder,
- Fig. 8: eine perspektivische Darstellung der isolierten Knochenschraube,
- Fig. 9: eine perspektivische Darstellung des isolierten Verbinders,
- Fig. 10: eine perspektivische Darstellung der isolierten Unterlegscheibe, und
- Fig. 11: eine perspektivische Darstellung der isolierten Fixiermutter.

In der Zeichnung ist eine Vorrichtung zur Stabilisierung der Wirbelsäule gezeigt, die ein Stab-Schrauben-System umfasst gebildet aus den Einzelteilen einer einen Schraubenschaft 2 aufweisenden Knochenschraube 1, einem Stab 3 und einem Verbinder 4.

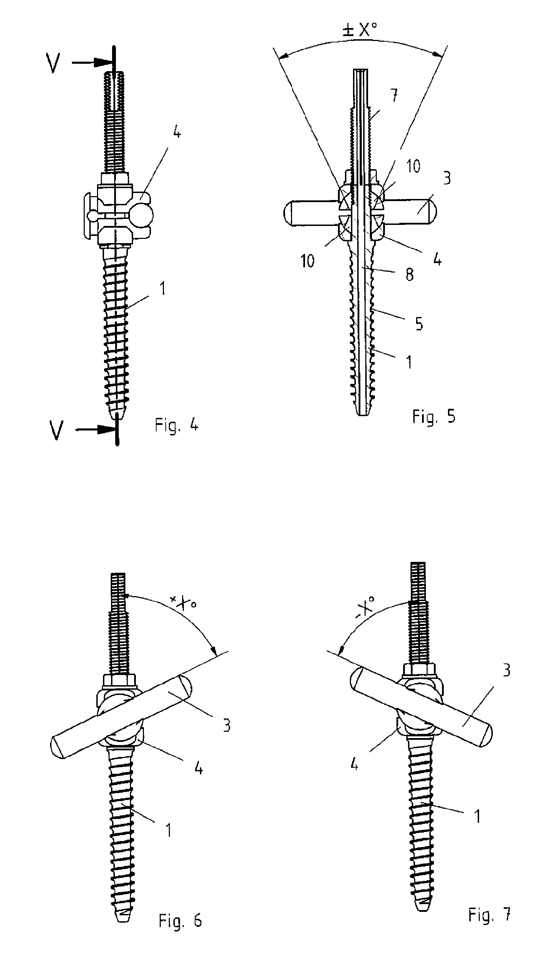

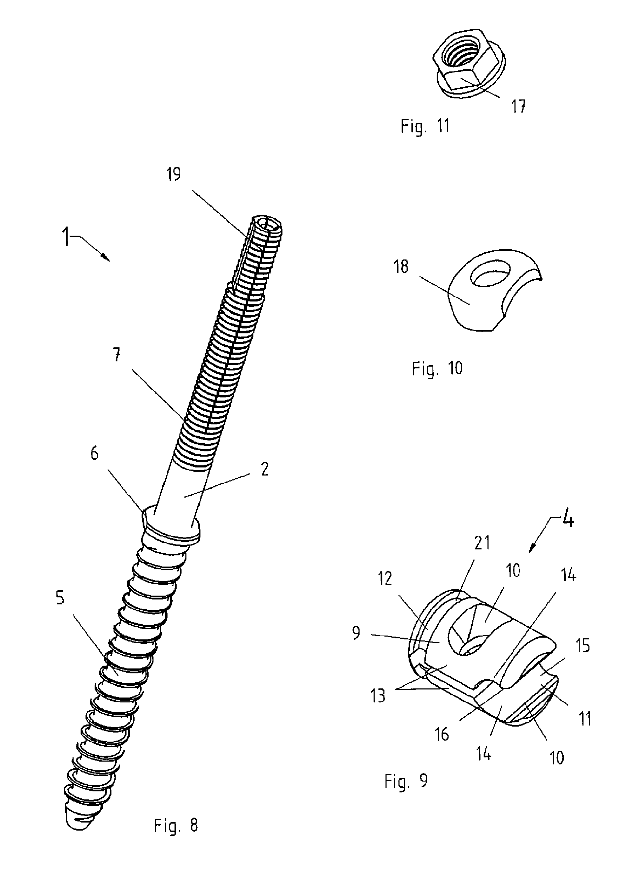

Figur 8 zeigt dabei isoliert die Knochenschraube 1, die einen zum Einbringen in den Knochen aufweisenden ersten Gewindeschnitte 5 aufweist. Benachbart zum ersten Gewindeschnitt 5 ist ein Schaftbund 6 und an dem dem ersten Gewindeabschnitt 5 gegenüberliegenden Ende des Schraubenschaftes 2 der Knochenschraube 1 ist ein zweiter Gewindeabschnitt 7 ausgebildet. Im Schraubenschaft verläuft zur Kanülierung ein Kanal 8 (Fig. 5)

Im Rahmen einer Operation zur Stabilisierung der Wirbelsäule ist es vorgesehen, eine Mehrzahl derartiger Knochenschrauben 1 in den Wirbelkörpern der Wirbelsäule mit dem ersten Gewindeabschnitt 5 einzubringen. Auf dem Schaftbund 6 der Knochenschraube 1 kann sodann der Verbinder 4 platziert werden, der einen Verbinderkörper 9 mit einer Durchgangsöffnung 10 und einer einseitig offenen Stabaufnahme 11 aufweist, in die unter Ausnutzung einer Federwirkung der Stab 3 eingeclipst werden kann. Ein entsprechender Verbinder ist isoliert in der Figur 9 dargestellt, die erkennen lässt, dass der Verbinderkörper 9 eine Körperbasis 12 und zwei daran mit gegenseitigem Abstand abgeschlossene Körperschenkel 13 aufweist, die an ihren freien Ende jeweils Körperstege 14 tragen, die den an die Umfangsgestalt des Stabes 3 angepassten Rastsitz 15 unter Ausnutzung jeweils mindestens einer am Körpersteg 14 angeschlossenen Stegleiste 16 bilden.

Die Figur 9 zeigt auch, dass die Durchgangsöffnung 10 im Verbinderkörper 9 als Langloch ausgebildet ist, das geneigt zur Längsachse des Verbinders 4 verlaufende Seitenwände aufweist, so dass sich die Möglichkeit ergibt, begrenzt durch die Seitenwände als Anschläge, den Verbinder 4 um seine Längsachse auf dem Schraubenschaft 2 zu verdrehen. Die entsprechende Justagemöglichkeit des Verbinders 4 relativ zu der Knochenschraube 1 ist in den Figuren 6 und 7 veranschaulicht. Nach der entsprechenden Positionierung des Verbinders 4 auf dem Schraubenschaft 2 der Knochenschraube 1 und der Ausrichtung des Verbinders 4 relativ zu der Knochenschraube 1 kann der Stab 3 in die Stabaufnahme 11 eingeclipst werden, wobei die so erzielte Konfiguration des Stab-Schrauben-Systems mittels einer auf dem zweiten Gewindeabschnitt 7 der Knochenschraube 1 aufzuschraubenden Fixiermutter 17 (Fig. 11), gegebenenfalls unter Zuhilfenahme einer Unterlegscheibe 18 (Fig. 10), fixiert werden kann. Die Unterlegscheibe 18besitzt eine an Verbinder 4 angepaßte und eine zur Anlage an die Fixiermutter 17 bestimmte zweite Seite. Unter Ausnutzung einer Sollbruchstelle oder eines im Bereich des zweiten Gewindeabschnitts 7 ausgebildete Schlitzen 19 kann der Schraubenschaft 2 gekürzt werden.

Durch die erfindungsgemäße Vorrichtung ergibt sich allerdings auch die Möglichkeit, dass vor der Positionierung des Stabes 3 in der Stabaufnahme 11 des Verbinders 4 dieser zunächst ausgenutzt wird, um eine korrekte Orientierung und Positionierung des Wirbelkörpers in der Wirbelsäule zu erzielen, wozu der Verbinder 4 als Koppelglied zum Anschließen eines Repositeurs 20 ausgebildet ist durch eine am Verbinderkörper 9 im Bereich der Körperbasis 12 in Umfangsrichtung verlaufende Ringnut 21. In diese Ringnut 21 kann der Repositeur 20 mit einer diesem zugeordneten Nut 22 aufgeschoben werden, der genutzt wird, um durch Anwendung entsprechender Hebelkräfte über die Knochenschraube 1 die Lageänderung des Wirbelkörpers zu bewirken. Nach der erfolgten Reponierung kann die so erzielte Ausrichtung des Wirbelkörpers mit der Knochenschraube 1 durch Anwendung des gegebenenfalls geeignet gebogenen Stabes 3 fixiert werden, so dass dann der Repositeur 20 von dem als Koppelglied wirkenden Verbinder 4 abgezogen werden kann.

### Bezugszeichenliste

- 1: Knochenschraube
- 2: Schraubenschaft
- 3: Stab
- 4: Verbinder
- 5: erster Gewindeabschnitt
- 6: Schaftbund
- 7: zweiter Gewindeabschnitt
- 8: Kanal
- 9: Verbinderkörper
- 10: Durchgangsöffnung
- 11: Stabaufnahme
- 12: Körperbasis
- 13: Körperschenkel
- 14: Körperstege
- 15: Rastsitz
- 16: Stegleiste
- 17: Fixiermutter
- 18: Unterlegscheibe
- 19: Schlitz
- 20: Repositeur
- 21: Ringnut
- 22: Nut

## Patentansprüche

1. Vorrichtung zur Stabilisierung der Wirbelsäule, mit einer einen Schraubenschaft (2) aufweisenden Knochenschraube (1), die einen zum Einbringen in den Knochen vorgesehenen ersten Gewindeabschnitt (5) aufweist, mit einem Stab (3) und mit einem an der Knochenschraube (1) befestigbaren Verbinder (4) zum lösbaren Verbinden des Stabes (3) mit der Knochenschraube (1), wobei der Verbinder (4) einen Verbinderkörper (9) mit einer Durchgangsöffnung (10) und einer einseitig offenen Stabaufnahme (11) aufweist, die durch zwei einander gegenüberliegende, zur Bildung eines Rastsitzes (15) jeweils mindestens eine Stegleiste (16) aufweisende Körperstege (14) gebildet ist, **dadurch gekennzeichnet, dass** die Stegleisten (16) die seitliche Öffnungsweite der Stabaufnahme (11) begrenzen derart, dass beim Einsclipsen des Stabes (3) in die Stabaufnahme (11) unter Ausnutzung einer Federwirkung zunächst eine Aufweitung der Stabaufnahme (11) mittels einer Auslenkung der Körperstege (14) erfolgt, und dass infolge der gewünschten Federwirkung die Körperstege (14) den Stab (3) nach der Einführung fest umschließen und einen an die Umfanggestalt des Stabes (3) angepassten Rastsitz bilden.

2. Vorrichtung nach Anspruch 1, **dadurch gekennzeichnet, dass** die Körperstege (14) an zwei mit gegenseitigem Abstand an einer Körperbasis (12) angeschlossenen Körperschenkeln (13) ausgebildet sind.

3. Vorrichtung nach Anspruche 1 oder 2, **dadurch gekennzeichnet, dass** die Durchgangsöffnung (10) als Langloch im Verbinderkörper (9) ausgebildet ist.

4. Vorrichtung nach Anspruch 3, wenn er von Anspruch 2 abhängig ist, **dadurch gekennzeichnet, dass** das Langloch in den Körperschenkeln (13) ausgebildet ist und geneigt zur Längsachse des Verbinders (4) verlaufende Seitenwände aufweist.

5. Vorrichtung nach einem der Ansprüche 1 bis 4, **dadurch gekennzeichnet, dass** der Verbinder (4) als Koppelglied zum Anschließen eines Repositeurs (20) ausgebildet ist.

6. Vorrichtung nach Anspruch 5, **dadurch gekennzeichnet, dass** der Verbinder (4) am Verbinderkörper (9) eine in Umfangsrichtung verlaufende Ringnut (21) zum Zusammenwirken mit dem Repositeur (20) aufweist.

7. Vorrichtung nach einem der Ansprüche 1 bis 6, **dadurch gekennzeichnet, dass** die Knochenschraube (1) benachbart zum ersten Gewindeabschnitt (5) einen Schaftbund (6) aufweist.

8. Vorrichtung nach einem der Ansprüche 1 bis 7, **dadurch gekennzeichnet, dass** am Schraubenschaft (2) ein zweiter Gewindeabschnitt (7) ausgebildet ist.

9. Vorrichtung nach Anspruch 8, **dadurch gekennzeichnet, dass** eine Fixiermutter (17) zum Zusammenwirken mit dem zweiten Gewindeabschnitt (7) vorgesehen ist.

10. Vorrichtung nach Anspruch 9, **dadurch gekennzeichnet, dass** eine Unterlegscheibe (18) mit einer an die Umfangsgestalt des Verbinderkörpers (9) angepassten einen Seite und einer zur Anlage an der Fixiermutter (17) bestimmten anderen Seite vorgesehen ist

11. Vorrichtung nach einem der Ansprüche 8 bis 10, **dadurch gekennzeichnet, dass** der Schraubenschaft (2) im Bereich des zweiten Gewindeabschnitts (7) mindestens einen den Schraubenschaft (2) in Schaftstege teilenden Schlitz (19) aufweist.

12. Vorrichtung nach einem der Ansprüche 8 bis 11, **dadurch gekennzeichnet, dass** im Bereich des zweiten Gewindeabschnitts (7) mindestens eine Sollbruchstelle ausgebildet ist.

13. Vorrichtung nach einem der Ansprüche 1 bis 12, **dadurch gekennzeichnet, dass** die Oberfläche der Stabaufnahme (11) und/oder des Stabes (3) die Haltewirkung erhöhende Strukturen und/oder Beschichtungen aufweist.

14. Kombination einer Vorrichtung zur Stabilisierung der Wirbelsäule nach einem der Ansprüche 1 bis 13, bestehend aus einer Knochenschraube (1), einem Stab (3) und einem Verbinder (4) mit einem Repositeur (20), **dadurch gekennzeichnet, dass** der Verbinder (4), als Koppelglied zum Abschließen des Repositeurs (20) ausgebildet ist und am Verbinderkörper (9) eine in Umfangsrichtung verlaufende Ringnut (21) zum Zusammenwirken mit einer einenends offenen Nut (22) des Repositeurs (20) aufweist.

## Claims

1. A device for stabilising the spinal column comprising a bone screw (1) which has a screw shank (2) and which has a first thread portion (5) intended for insertion into the bone, a rod (3) and a connector (4) which can be fixed to the bone screw (1) for releasably connecting the rod (3) to the bone screw (1), the connector (4) having a connector body (9) with a through opening (10) and a rod receiving means (11) which is open at one side and which is formed by two mutually opposite body legs (14) each having at least one respective leg bar (16) for forming a latching seat (15), **characterised in that** the leg bars (16) delimit the lateral opening width of the rod receiving means (11) in such a way that when the rod (3) is clipped into the rod receiving means (11), enlargement of the rod receiving means (11) occurs by means of a deflection of the body legs (14) by utilising a spring action and that as a result of the desired spring action the body legs (14) fixedly embrace the rod (3) after introduction thereof and form a latching seat adapted to the peripheral configuration of the rod (3).

2. A device according to claim 1 **characterised in that** the body legs (14) are provided on two body limbs (13) connected at a mutual spacing to a body base (12).

3. A device according to claim 1 or claim 2 **characterised in that** the through opening (10) is in the form of a slot in the connector body (9).

4. A device according to claim 3 when appended to claim 2 **characterised in that** the slot is provided in the body limbs (13) and has side walls extending inclinedly relative to the longitudinal axis of the connector (4).

5. A device according to one of claims 1 to 4 **characterised in that** the connector (4) is in the form of a coupling member for connecting a repositor (20).

6. A device according to claim 5 **characterised in that** the connector (4) on the connector body (9) has an annular groove (21) extending in the peripheral direction for co-operation with the repositor (20).

7. A device according to one of claims 1 to 6 **characterised in that** the bone screw (1) has a shank collar (6) adjacent to the first thread portion (5).

8. A device according to one of claims 1 to 7 **characterised in that** a second thread portion (7) is provided on the screw shank (2).

9. A device according to claim 8 **characterised in that** there is provided a fixing nut (17) for co-operating with the second thread portion (7).

10. A device according to claim 9 **characterised in that** there is provided a washer (18) having a one side adapted to the peripheral configuration of the connector body (9) and another side intended for bearing against the fixing nut (17).

11. A device according to one of claims 8 to 10 **characterised in that** in the region of the second thread portion (7) the screw shank (2) has at least one slot (19) dividing the screw shank (2) into shank legs.

12. A device according to one of claims 8 to 11 **characterised in that** at least one desired-fracture location is provided in the region of the second thread portion (7).

13. A device according to one of claims 1 to 12 **characterised in that** the surface of the rod receiving mans (11) and/or of the rod (3) has coatings and/or structures enhancing the holding effect.

14. A combination of a device for stabilising the spinal column according to one of claims 1 to 13 comprising a bone screw (1), a rod (3) and a connector (4) having a repositor (20), **characterised in that** the connector (4) is in the form of a coupling member for connection of the repositor (20) and on the connector body (9) has an annular groove (21) extending in the peripheral direction for co-operation with a groove (22) of the repositor (20), the groove (22) being open at one end.

## Revendications

1. Dispositif de stabilisation de la colonne vertébrale, comportant une vis à os (1) dotée d'une tige de vis (2) et présentant une première section filetée (5) prévue pour être introduite dans les os, une barre (3) et une attache (4) pouvant être fixée à la vis à os (1) en vue de la liaison amovible de la barre (3) avec la vis à os (1), l'attache (3) comprenant un corps d'attache (9) présentant un orifice de passage (10) et un logement de barre (11) ouvert d'un côté et formé par deux ailes de corps (14) qui présentent chacune un rebord d'aile (16) et qui sont situées l'une en face de l'autre pour former un siège d'encliquetage (15), **caractérisé en ce que** les rebords d'aile (16) limitent la largeur d'ouverture latérale du logement de barre (11), de façon que, lorsque la barre (3) est clipsée dans le logement de barre (11), il se produise d'abord, en utilisant un effet élastique, un élargissement du logement de barre (11) moyennant une déviation des ailes de corps (14), et que, par suite de l'effet de ressort souhaité, les ailes de corps (14) enserrent fermement la barre (3) après son introduction et forment un siège d'encliquetage épousant la forme périphérique de la barre (3).

2. Dispositif selon la revendication 1, **caractérisé en ce que** les ailes de corps (14) sont réalisées sur deux branches de corps (13) raccordées à distance l'une de l'autre à une base de corps (12).

3. Dispositif selon la revendication 1 ou 2, **caractérisé en ce que** l'orifice de passage (10) est réalisé sous la forme d'un trou oblong dans le corps d'attache (9).

4. Dispositif selon la revendication 3, si elle dépend de la revendication 2, **caractérisé en ce que** le trou oblong est ménagé dans les branches de corps (13) et présente des parois latérales inclinées par rapport à l'axe longitudinal de l'attache (4).

5. Dispositif selon l'une des revendications 1 à 4, **caractérisé en ce que** l'attache (4) est réalisée sous la forme d'un organe d'accouplement servant à raccorder un repositionneur (20).

6. Dispositif selon la revendication 5, **caractérisé en ce que** l'attache (4) présente sur le corps d'attache (9) une rainure annulaire (21) qui s'étend en direction périphérique et qui est conçue pour coopérer avec le repositionneur (20).

7. Dispositif selon l'une des revendications 1 à 6, **caractérisé en ce que** la vis à os (1) présente une collerette de tige (6) adjacente à la première section filetée (5).

8. Dispositif selon l'une des revendications 1 à 7, **caractérisé en ce qu'**une seconde section filetée (7) est réalisée sur la tige de vis (2).

9. Dispositif selon la revendication 8, **caractérisé en ce qu'**un écrou de fixation (17) destiné à coopérer avec la seconde section filetée (7) est prévu.

10. Dispositif selon la revendication 9, **caractérisé en ce qu'**une rondelle plate (18) avec un côté épousant la forme périphérique du corps d'attache (9) et un autre côté destiné à être appliqué contre l'écrou de fixation (17) est prévu.

11. Dispositif selon l'une des revendications 8 à 10, **caractérisé en ce que**, dans la zone de la seconde section filetée (7), la tige de vis (2) présente au moins une fente (19) divisant la tige de vis (2) en ailes de tige.

12. Dispositif selon l'une des revendications 8 à 11, **caractérisé en ce qu'**au moins une zone destinée à la rupture est réalisée dans la zone de la seconde section filetée (7).

13. Dispositif selon l'une des revendications 1 à 12, **caractérisé en ce que** la surface du logement de barre (11) et/ou de la barre (3) présente des structures et/ou des revêtements augmentant l'effet de retenue.

14. Combinaison d'un dispositif de stabilisation de la colonne vertébrale selon l'une des revendications 1 à 13, composée d'une vis à os (1), d'une barre (3) et d'une attache (4) avec un repositionneur (20), **caractérisé en ce que** l'attache (4) est réalisée sous la forme d'un organe d'accouplement servant à raccorder le repositionneur (20) et présente, sur le corps d'attache (9), une rainure annulaire (21) qui s'étend en direction périphérique et qui est conçue pour coopérer avec une rainure (22) ouverte d'un côté du repositionneur (20).
